(19) Europäisches Patentamt
European Patent Office
Office européen des brevets

(11) Veröffentlichungsnummer: **0 315 992 B1**

(12) # EUROPÄISCHE PATENTSCHRIFT

(45) Veröffentlichungstag der Patentschrift: **11.08.93**

(51) Int. Cl.[5]: **C07C 317/14**, C07D 209/49

(21) Anmeldenummer: **88118751.2**

(22) Anmeldetag: **10.11.88**

Verbunden mit 88909751.5/0390803
(europäische
Anmeldenummer/Veröffentlichungsnummer)
durch Entscheidung vom 11.04.91.

(54) **Schwefelhaltige aromatische Tetracarbonsäuren und deren Derivate.**

(30) Priorität: **12.11.87 DE 3738456**

(43) Veröffentlichungstag der Anmeldung:
**17.05.89 Patentblatt 89/20**

(45) Bekanntmachung des Hinweises auf die Patenterteilung:
**11.08.93 Patentblatt 93/32**

(84) Benannte Vertragsstaaten:
**AT BE CH DE ES FR GB GR IT LI LU NL SE**

(56) Entgegenhaltungen:
**EP-A- 0 210 036**
**EP-A- 0 245 815**
**US-A- 3 895 064**
**US-A- 4 255 313**

**K. Bühler, Spezialplaste, Akademie-Verlag Berlin, 1978, S. 600, 601**

**J. POLYMER SCI., A, vol. 1, 1963, S. 3139**

(73) Patentinhaber: **Chemie Linz Gesellschaft m.b.H.**
**St.Peter-Strasse 25**
**A-4021 Linz(AT)**

(72) Erfinder: **Greber, Gerd**
**Anzengruberstrasse 11**
**A-2540 Bad Vöslau(AT)**
Erfinder: **Gruber, Heinrich**
**Cottagegasse 82**
**A-1190 Wien(AT)**
Erfinder: **Sychra, Marcel**
**Friedhofstrasse 44/11**
**A-2103 Langenzersdorf(AT)**

(74) Vertreter: **Kunz, Ekkehard, Dr.**
**Chemie Holding AG Patentwesen St. Peter-Strasse 25**
**A-4021 Linz (AT)**

EP 0 315 992 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung beschreibt neue schwefelhaltige, aromatische Tetracarbonsäuren und deren Derivate sowie Verfahren zu deren Herstellung.

Tetracarbonsäuredianhydride, wie z. B. Pyromellithsäuredianhydrid oder Benzophenontetracarbonsäuredianhydrid, werden in der Technik beispielsweise zur Herstellung von besonders thermostabilen Polymeren, den Polyimiden, eingesetzt. Aromatische Polyimide können z. B. in 2 Stufen, durch Polyaddition und Polykondensation, hergestellt werden. In der ersten Stufe wird ein aromatisches Diamin mit einem aromatischen Tetracarbonsäuredianhydrid zur Polyamidsäure polyaddiert. In dieser Form sind die Polymere noch gut löslich und können z. B. zu Folien und Fasern verarbeitet werden. In der zweiten Stufe erfolgt durch thermische oder chemische Wasserabspaltung die intramolekulare Cyclisierung zum unlöslichen, hochwärmebeständigen Polyimid (Macromol.Revs., 11 (1976) 161-208). Da in geringem Umfang auch schon bei Raumtemperatur die Umwandlung der Polyamidsäure in das unlösliche Polyimid erfolgt, und somit mit zunehmender Zeitdauer der Anteil der löslichen Polyamidsäure abnimmt, sind die Polyamidsäurelösungen nur begrenzt lagerfähig.

Die ausschließlich aus aromatischen Bausteinen bestehenden Polyimide sind zwar extrem thermostabil, aber völlig unlöslich und unschmelzbar und somit nur schwer verarbeitbar (J.Polym.Sci., A3 (1965) 1373-1390). Diese schwierige Verarbeitbarkeit verteuert die Polyimide ganz erheblich und schließt sie daher aus einer Reihe von interessanten Anwendungen aus.

Durch Veränderungen der chemischen Struktur der aromatischen Grundbausteine, z. B. durch Einbau von flexiblen Kettenelementen ($-CH_2-$, -O-, -S-, -CO-) oder sterisch anspruchsvollen Gruppen, konnten lösliche und/oder schmelzbare und damit einfacher verarbeitbare Polyimide erhalten werden, die allerdings generell eine geringere thermische Stabilität aufweisen (J.Appl.Polym.Sci., 26 (1981) 3837-3843). Die Polyamidimide, die beispielsweise aus aromatischen Tricarbonsäuremonoanhydriden und aromatischen Diaminen hergestellt werden, stellen Produkte mit vergleichbarem Eigenschaftsprofil dar (US-PS 3 895 064, US-PS 4 724 257). Auch sie sind in polaren organischen Lösungsmitteln löslich und zeigen meist thermoplastische Eigenschaften, besitzen aber im Vergleich zu den aromatischen Polyimiden ebenfalls eine geringere Thermostabilität.

Aufgabe der vorliegenden Erfindung war es, Tetracarbonsäuren oder deren Derivate zu entwickeln, aus denen beispielsweise durch Polyaddition mit geeigneten Diaminen und anschließende Cyclisierung Polyimide bzw. Polyamidimide hergestellt werden können, die bei extremer Thermostabilität in der cyclisierten Form löslich und/oder schmelzbar, und somit nach herkömmlichen Methoden einfach verarbeitbar sind.

Es wurde nun gefunden, daß diese Aufgabe mit Hilfe von Tetracarbonsäuren oder deren Derivaten, die -S- und $-SO_2-$Brücken enthaltende, aromatische, flexible Kettenelemente aufweisen, gelöst werden kann.

Gegenstand der vorliegenden Erfindung sind demnach aromatische Tetracarbonsäuren oder deren Derivate der allgemeinen Formel I des Formelblattes, worin

- R einen divalenten Rest der Formel II des Formelblattes,
- Ar tri- oder tetravalente, gegebenenfalls ein- oder mehrfach durch Halogen substituierte aromatische Reste oder Gemische derselben, wobei der trivalente aromatische Rest gegebenenfalls zusätzlich eine Carboxylgruppe als Substituenten enthalten kann,
- X falls Ar trivalent ist, den Rest -CO-NH- und falls Ar tetravalent ist, den Imidrest der Formel III des Formelblattes, wobei jeweils N an R gebunden ist,
- Y,Z, entweder gemeinsam den Anhydridrest -CO-O-CO-, oder für sich allein und unabhängig voneinander die Reste -COOH, -COCl oder $-COOR_1$, wobei $R_1$ einen Alkylrest mit 1 - 20 C-Atomen oder einen Arylrest mit 6 bis 20 C-Atomen darstellt,

bedeuten.

Erfindungsgemäß kommen alle bekannten tri- oder tetravalenten aromatischen Reste Ar in Frage, die sich insbesondere von den entsprechenden Tri- oder Tetracarbonsäuren bzw. ihren Derivaten, wie beispielsweise Anhydriden, Estern oder Säurechloriden ableiten. Beispiele dafür sind Reste von Aromaten, kondensierten Aromaten, Heteroaromaten, sowie deren Derivaten.

Bevorzugt sind aromatische Tetracarbonsäuren oder deren Derivate der allgemeinen Formel I des Formelblattes, in denen als aromatischer Rest Ar ein Benzol- oder Benzophenonrest enthalten ist. Die Aromaten können auch substituiert sein.

Falls der trivalente Rest Ar zusätzlich eine Carboxylgruppe als Substituenten enthält, sich also von einer Tetracarbonsäure oder deren Derivaten ableitet, dann liegt das erfindungsgemäße Tetracarbonsäurederivat der allgemeinen Formel I des Formelblattes in Form einer Bis-Amidsäure entsprechend der allgemeinen Formel VII des Formelblattes vor, worin Y und Z die obige Bedeutung haben. Im Falle der erfindungsgemäßen aromatischen Tetracarbonsäureester der allgemeinen Formel I des Formelblattes stellt $R_1$ bevorzugt

einen niederen Alkylrest mit 1 bis 8 C-Atomen bzw. einen Benzolrest dar.

Die erfindungsgemäßen aromatischen Tetracarbonsäuren oder deren Derivate können dadurch hergestellt werden, daß man Tricarbonsäuremonoanhydridchloride oder Tetracarbonsäuredianhydride oder deren Gemische der allgemeinen Formel IV des Formelblattes, in der im Falle der Tricarbonsäuremonoanhydridchloride $R_2$ für H und $R_3$ für -COCl und im Falle der Tetracarbonsäuredianhydride $R_2$ und $R_3$ gemeinsam für den Anhydridrest -CO-O-CO- stehen, mit dem Diamin der allgemeinen Formel V des Formelblattes (Herstellung beispielsweise gemäß EP-A-0 245 815), worin R die obige Bedeutung hat, im Molverhältnis von mindestens 2 : 1 in an sich bekannter Weise umsetzt, wobei im Falle der Verwendung der Tricarbonsäuremonoanhydridchloride der allgemeinen Formel IV des Formelblattes die Tetracarbonsäuredianhydride der Formel I des Formelblattes, in der X den Rest -CO-NH-bedeutet, und im Falle der Verwendung der Tetracarbonsäuredianhydride der allgemeinen Formel IV des Formelblattes zunächst die Bis-Amidsäuredianhydride der allgemeinen Formel VI des Formelblattes, entstehen, die gegebenenfalls chemisch oder thermisch zu den Tetracarbonsäuredianhydriden der allgemeinen Formel I des Formelblattes , in der X den Imidrest der Formel III des Formelblattes bedeutet, cyclisiert werden, worauf die gebildeten Tetracarbonsäuredianhydride der allgemeinen Formel I gegebenenfalls in an sich bekannter Weise mit Wasser, Alkoholen und/oder Salzsäure ganz oder teilweise zu den entsprechenden Säuren, Säurechloriden oder Estern der allgemeinen Formel I umgesetzt werden.

Die Umsetzung der Tri- bzw. Tetracarbonsäurederivate der allgemeinen Formel IV des Formelblattes mit dem Diamin der allgemeinen Formel V des Formelblattes kann in an sich bekannter Weise in stark polaren Lösungsmitteln, wie z. B. Dimethylformamid (DMF), Dimethylacetamid (DMA), N-Methylpyrrolidon (NMP), Dimethylsulfoxid (DMSO), Tetramethylharnstoff oder Hexamethylphosphorsäuretriamid erfolgen. Zweckmäßigerweise gibt man bei den Umsetzungen der Tricarbonsäuremonoanhydridchloride einen HCl-Fänger, beispielsweise ein tertiäres Amin zu.

Die Isolierung der erfindungsgemäßen Tetracarbonsäuren oder deren Derivaten kann entweder durch Abdestillieren des Lösungsmittels (beim Ansatz 1:1) oder durch Ausfällen (z. B. in Benzol) mit anschließendem Umkristallisieren (z. B. aus Acetanhydrid) erfolgen.

Setzt man die beiden Partner exakt im stöchiometrischen Verhältnis 2 : 1 ein, dann können die intermediär entstehenden Lösungen der Tetracarbonsäuren bzw. deren Derivaten der allgemeinen Formel I des Formelblattes auch ohne isoliert zu werden für weitere Reaktionen - z. B. Polyreaktionen mit aromatischen Diaminen - verwendet werden.

Die Reaktionstemperatur kann je nach verwendeten Ausgangsstoffen und gewünschtem Endprodukt innerhalb eines weiten Bereiches schwanken. Bei Verwendung der Anhydridchloride, wie z. B. Trimellithsäureanhydridchlorid, liegen die Reaktionstemperaturen zwischen -50 und +30 °C, bei Verwendung der Tetracarbonsäurederivate zwischen 0 und +130 °C, wobei nach Beendigung der Reaktion die zunächst resultierenden Bis-Amidsäuren, zweckmäßig ohne Isolierung, beispielsweise chemisch cyclisiert werden, z. B. mittels Acetanhydrid bei Temperaturen zwischen 100 und 150 °C.

Zur Synthese der erfindungsgemäßen Tetracarbonsäuren oder deren Derivaten werden bevorzugt Benzophenontetracarbonsäuredianhydrid, Pyromellithsäuredianhydrid oder Trimellithsäureanhydridchlorid eingesetzt. Erfindungsgemäß können aber auch andere Tetracarbonsäuredianhydride, so z. B.

4,4′, 5,5′, 6,6′-Hexafluorbenzophenon-2,2′, 3,3′-tetracarbonsäuredianhydrid
3,3′, 4,4′-Diphenyl-tetracarbonsäuredianhydrid
2,2′, 3,3′-Diphenyl-tetracarbonsäuredianhydrid
Bis(2,3-dicarboxyphenyl)methan-dianhydrid
Bis(3,4-dicarboxyphenyl)methan-dianhydrid
2,2-Bis(3,4-dicarboxyphenyl)propan-dianhydrid
2,2-Bis(2,3-dicarboxyphenyl)propan-dianhydrid
Bis(3,4-dicarboxyphenyl)äther-dianhydrid
Bis(2,3-dicarboxyphenyl)äther-dianhydrid
Bis(3,4-dicarboxyphenyl)sulfon-dianhydrid
Bis(3,4-dicarboxyphenyl)phenylphosphonat-dianhydrid
3,3′, 4,4′-Tetracarboxybenzoyloxybenzol-dianhydrid
1,4,5,8-Naphthalin-tetracarbonsäuredianhydrid
2,3,6,7-Naphthalin-tetracarbonsäuredianhydrid
1,2,5,6-Naphthalin-tetracarbonsäuredianhydrid
2,3,6,7-Tetrachlornaphthalin-1,4,5,8-tetracarbonsäuredianhydrid
Phenanthren-1,8,9,10-tetracarbonsäuredianhydrid
3,4,9,10-Perylen-tetracarbonsäuredianhydrid
Thiophen-2,3,4,5-tetracarbonsäuredianhydrid

Pyrazin-2,3,5,6-tetracarbonsäuredianhydrid
Pyridin-2,3,5,6-tetracarbonsäuredianhydrid
mit dem Diamin der Formel V des Formelblattes umgesetzt werden.

Anstelle von Trimellithsäureanhydridchlorid können auch andere bekannte Tricarbonsäuremonoanhydridchloride, so z. B.
4-(4-(Chlorcarbonyl)phenoxy)phthalsäureanhydrid
4-(4-(Chlorcarbonyl)benzoyl)phthalsäureanhydrid
4-(4-(Chlorcarbonyl)phenylsulfonyl)phthalsäureanhydrid
4-(4-(Chlorcarbonyl)benzyl)phthalsäureanhydrid
mit dem Diamin der Formel V des Formelblattes umgesetzt werden.

Beispiel 1:

Umsetzung von 1,4-Bis(4-aminophenylthio)diphenylsulfon (BDS) mit Trimellithsäureanhydridchlorid (TMACl)

Ansatz:

10,85 g (0,05 Mol) TMACl
9,30 g (0,02 Mol) BDS
90 ml abs. N-Methyl-2-Pyrrolidon (NMP)
Die Apparatur, bestehend aus einem 250 ml-Dreihalskolben mit mechanischem Rührer, Thermometer, Tropftrichter mit Trockenrohr und Stickstoffeinleitungsrohr wird im trockenen Stickstoffstrom ausgeflammt. TMACl wird in 25 ml NMP gelöst, mit Trockeneis/Aceton auf -40 °C abgekühlt und bei dieser Temperatur BDS in 65 ml NMP während 30 Min. zugetropft. Das Reaktionsgemisch wird nach weiteren 30 Min. bei -40 °C langsam auf Raumtemperatur erwärmt und anschließend noch 3 Stunden gerührt.
Die erhaltene viskose Lösung wird unter Ausschluß von Luftfeuchtigkeit unter Rühren bei 10 °C in 500 ml abs. Benzol eingetropft, bis nach etwa 1 Stunde ein hellgelber Niederschlag ausfällt, der abfiltriert, mit kaltem und heißem abs. Benzol gewaschen und anschließend bei 60 °C im Vakuumtrockenschrank getrocknet wird. Das Rohprodukt wird aus abs. Acetanhydrid umkristallisiert.
Ausbeute: 14,7 g (90 % d. Th.), Schmelzpunkt: 282 - 288 °C

| Analyse: | berechnet (%) | gefunden (%) |
|---|---|---|
| $C_{42}$ | 62,05 | 61,62 |
| $H_{24}$ | 2,98 | 3,16 |
| $N_2$ | 3,45 | 3,56 |
| $S_3$ | 11,83 | 12,24 |
| $O_{10}$ | - | - |

Beispiel 2:

Umsetzung von BDS mit Benzophenontetracarbonsäuredianhydrid (BTDA)

Ansatz:

6,4446 g (0,02 Mol) BTDA
4,6464 g (O,01 Mol) BDS
3,6 g (0,035 Mol) Acetanhydrid
60 ml abs. Dimethylacetamid (DMA)
In einer trockenen Apparatur, bestehend aus einem 100 ml-Dreihalskolben, Magnetrührer, Rückflußkühler, Tropftrichter, Stickstoffeinleitungsrohr, Thermometer und Trockenrohr wird das BTDA in 25 ml DMA gelöst. BDS in 35 ml DMA gelöst, wird während 15 Min. bei Raumtemperatur zugetropft und das Reaktionsgemisch auf 90 °C erhitzt. Nach 30 Min. werden 3,6 g Acetanhydrid zugegeben und die Temperatur für 1 Stunde auf 125 °C erhöht. Die dunkelbraune Lösung wird einrotiert, aus abs. Acetanhydrid umkristallisiert und anschließend im Vakuumtrockenschrank bei 90 °C getrocknet.
Ausbeute: 9,5 g (88 % d. Th.), Schmelzpunkt 238 - 242 °C

| Analyse: | berechnet (%) | gefunden (%) |
|---|---|---|
| $C_{58}$ | 64,92 | 64,37 |
| $H_{28}$ | 2,64 | 2,92 |
| $N_2$ | 2,61 | 2,39 |
| $S_3$ | 8,96 | 8,76 |
| $O_{14}$ | - | - |

Beispiel 3:

Veresterung des im Beispiel 2 erhaltenen Dianhydrides

Ansatz:

2,15 g (0,002 Mol) Dianhydrid
20 ml abs. Butanol
10 ml abs. Pyridin
25 ml abs. DMA

Das Dianhydrid wird in einem Gemisch von Butanol, Pyridin und DMA unter Rückfluß gekocht. Das Reaktionsgemisch wird nach 6 Stunden auf 5 %ige NaCl-Lösung gegossen, der ausgefallene Ester abfiltriert und bei 60 °C im Vakuumtrockenschrank getrocknet.
Ausbeute: 2,11 g (89 % d. Th.), Schmelzpunkt: 273 - 278 °C

| Analyse: | berechnet (%) | gefunden (%) |
|---|---|---|
| $C_{66}$ | 64,90 | 64,63 |
| $H_{48}$ | 3,97 | 4,28 |
| $N_2$ | 2,29 | 2,06 |
| $S_3$ | 7,87 | 7,54 |
| $O_{16}$ | - | - |

Beispiel 4:

Umsetzung von BDS mit Pyromellithsäuredianhydrid (PMDA)

Ansatz:

9,597 g (0,044 Mol) PMDA
9,293 g (0,02 Mol) 1,4-Bis(4-aminophenylthio)diphenylsulfon (BDS)
7,2 g (0,07 Mol) abs. Acetanhydrid
100 ml abs. DMA

In einer trockenen Apparatur, bestehend aus einem 250 ml-Dreihalskolben, Magnetrührer, Rückflußkühler, Tropftrichter, Stickstoffeinleitungsrohr, Thermometer und Trockenrohr wird das PMDA in 35 ml DMA gelöst. BDS in 65 ml DMA gelöst, wird während 15 Min bei Raumtemperatur zugetropft und das Reaktionsgemisch auf 90 °C erhitzt. Nach 30 Min werden 7,2 g Acetanhydrid zugegeben und die Temperatur für 1 Stunde auf 125 °C erhöht. Die erhaltene viskose Lösung wird unter Ausschluß von Luftfeuchtigkeit unter Rühren bei 10 °C in 500 ml abs. Benzol eingetropft, der ausgefallene gelbe Niederschlag abfiltriert, mit kaltem und heißem abs. Benzol gewaschen und anschließend bei 60 °C im Vakuumtrockenschrank getrocknet. Das Rohprodukt wird aus abs. Acetanhydrid umkristallisiert.
Ausbeute: 14,4 g (83 % d. Th.), Schmelzpunkt: 256 - 261 °C

| Analyse: | berechnet ( % ) | gefunden ( % ) |
|---|---|---|
| $C_{44}$ | 61,11 | 60,83 |
| $H_{20}$ | 2,33 | 2,57 |
| $N_2$ | 3,24 | 3,03 |
| $S_3$ | 11,12 | 10,98 |
| $O_{12}$ | - | - |

Beispiel 5:

Hydrolyse des im Beispiel 4 erhaltenen Dianhydrides

Ansatz:

1,73 g (0,002 Mol) Dianhydrid
100 ml 0,01 n HCl
Das Dianhydrid wird 4 Stunden unter Rückfluß in 0,01 n HCl gekocht. Die erhaltene Tetracarbonsäure wird abfiltriert und im Vakuumtrockenschrank bei 70 ° C getrocknet.
Ausbeute: 1,74 g (97 % d. Th.), Schmelzpunkt: 248 - 252 ° C

| Analyse: | berechnet (%) | gefunden (%) |
|---|---|---|
| $C_{44}$ | 58,79 | 58,52 |
| $H_{24}$ | 2,47 | 2,79 |
| $N_2$ | 3,12 | 2,86 |
| $S_3$ | 10,70 | 10,42 |
| $O_{14}$ | - | - |

**Patentansprüche**
**Patentansprüche für folgende Vertragsstaaten : AT, BE, CH, DE, FR, GB, GR, IT, LI, LU, NL, SE**

**1.** Aromatische Tetracarbonsäuren oder deren Derivate der allgemeinen Formel I

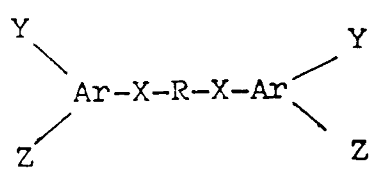

(I)

worin

R einen divalenten Rest der Formel II

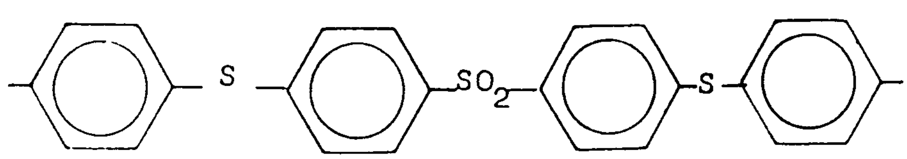

(II)

Ar tri- oder tetravalente, gegebenenfalls ein- oder mehrfach durch Halogen substituierte aromatische Reste oder Gemische derselben, wobei der trivalente aromatische Rest gegebenenfalls zusätzlich eine Carboxylgruppe als Substituenten enthalten kann,

X falls Ar trivalent ist, den Rest -CO-NH- und falls Ar tetravalent ist, den Imidrest der Formel III

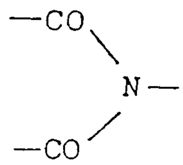

(III)

wobei jeweils N an R gebunden ist,

Y,Z, entweder gemeinsam den Anhydridrest -CO-O-CO-, oder für sich allein und unabhängig voneinander die Reste -COOH, -COCl oder $-COOR_1$, wobei

$R_1$ einen Alkylrest mit 1 bis 20 C-Atomen oder einen Arylrest mit 6 bis 20 C-Atomen darstellt,

bedeuten.

**2.** Aromatische Tetracarbonsäuren oder deren Derivate gemäß Anspruch 1, dadurch gekennzeichnet, daß der Rest Ar einen Benzophenonrest darstellt.

**3.** Aromatische Tetracarbonsäuren oder deren Derivate gemäß Anspruch 1 oder 2, dadurch gekennzeichnet, daß der Rest Ar einen Benzolrest darstellt.

**4.** Verfahren zur Herstellung von aromatischen Tetracarbonsäuren oder deren Derivaten gemäß einem der Ansprüche 1 bis 3, dadurch gekennzeichnet, daß man Tricarbonsäuremonoanhydridchloride oder Tetracarbonsäuredianhydride oder deren Gemische der allgemeinen Formel IV

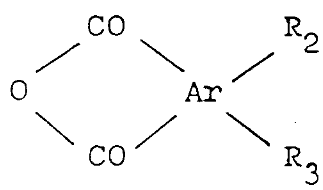

(IV)

in der im Falle der Tricarbonsäuremonoanhydridchloride $R_2$ für H und $R_3$ für - COCl und im Falle der Tetracarbonsäuredianhydride $R_2$ und $R_3$ gemeinsam für den Anhydridrest -CO-O-CO- stehen, mit dem Diamin der allgemeinen Formel V

$H_2N-R-NH_2$ (V)

worin R die obige Bedeutung hat, im Molverhältnis von mindestens 2 : 1 in an sich bekannter Weise umsetzt, wobei im Falle der Verwendung der Tricarbonsäuremonoanhydridchloride der allgemeinen Formel IV die Tetracarbonsäuredianhydride der Formel I in der X den Rest -CO-NH-bedeutet, und im Falle der Verwendung der Tetracarbonsäuredianhydride der allgemeinen Formel IV zunächst die Bis-Amidsäuredianhydride der allgemeinen Formel VI

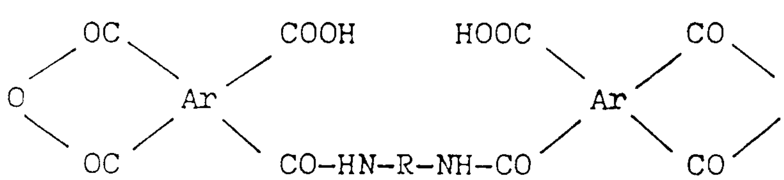

(VI)

entstehen, die gegebenenfalls chemisch oder thermisch zu den Tetracarbonsäuredianhydriden der allgemeinen Formel I in der X den Imidrest der Formel III bedeutet, cyclisiert werden, worauf die gebildeten Tetracarbonsäuredianhydride der allgemeinen Formel I gegebenenfalls in an sich bekannter Weise mit Wasser, Alkoholen und/oder Salzsäure ganz oder teilweise zu den entsprechenden Säuren, Säurechloriden oder Estern der allgemeinen Formel I umgesetzt werden.

5. Verfahren gemäß Anspruch 4, dadurch gekennzeichnet, daß man als Tricarbonsäuremonoanhydridchlorid Trimellithsäureanhydridchlorid einsetzt.

6. Verfahren gemäß Anspruch 4 oder 5, dadurch gekennzeichnet, daß man als Tetracarbonsäuredianhydrid Pyromellithsäuredianhydrid einsetzt.

7. Verfahren gemäß einem der Ansprüche 4 bis 6, dadurch gekennzeichent, daß man als Tetracarbonsäuredianhydrid Benzophenontetracarbonsäuredianhydrid einsetzt.

**Patentansprüche für folgenden Verstragsstaat : ES**

1. Verfahren zur Herstellung von aromatische Tetracarbonsäuren oder deren Derivaten der allgemeinen Formel I

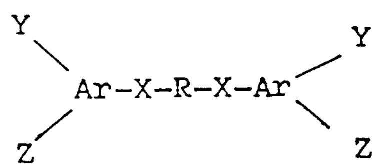

(I)

worin

R einen divalenten Rest der Formel II

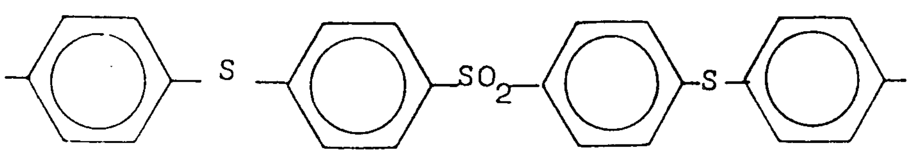

(II)

Ar tri- oder tetravalente, gegebenenfalls ein- oder mehrfach durch Halogen substituierte aromatische Reste oder Gemische derselben, wobei der trivalente aromatische Rest gegebenenfalls zusätzlich eine Carboxylgruppe als Substituenten enthalten kann,

X falls Ar trivalent ist, den Rest -CO-NH- und falls Ar tetravalent ist, den Imidrest der Formel III

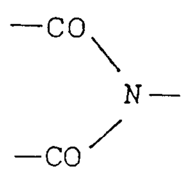

(III)

wobei jeweils N an R gebunden ist,

Y,Z, entweder gemeinsam den Anhydridrest -CO-O-CO-, oder für sich allein und unabhängig voneinander die Reste -COOH, -COCl oder -COOR$_1$, wobei

R$_1$ einen Alkylrest mit 1 bis 20 C-Atomen oder einen Arylrest mit 6 bis 20 C-Atomen darstellt,

bedeuten, dadurch gekennzeichnet, daß man Tricarbonsäuremonoanhydridchloride oder Tetracarbonsäuredianhydride oder deren Gemische der allgemeinen Formel IV

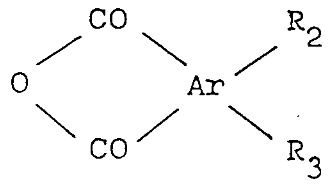

(IV)

in der im Falle der Tricarbonsäuremonoanhydridchloride $R_2$ für H und $R_3$ für - COCl und im Falle der Tetracarbonsäuredianhydride $R_2$ und $R_3$ gemeinsam für den Anhydridrest -CO-O-CO- stehen, mit dem Diamin der allgemeinen Formel V

$H_2N$-R-$NH_2$ (V)

worin R die obige Bedeutung hat, im Molverhältnis von mindestens 2 : 1 in an sich bekannter Weise umsetzt, wobei im Falle der Verwendung der Tricarbonsäuremonoanhydridchloride der allgemeinen Formel IV die Tetracarbonsäuredianhydride der Formel I in der X den Rest -CO-NH-bedeutet, und im Falle der Verwendung der Tetracarbonsäuredianhydride der allgemeinen Formel IV zunächst die Bis-Amidsäuredianhydride der allgemeinen Formel VI

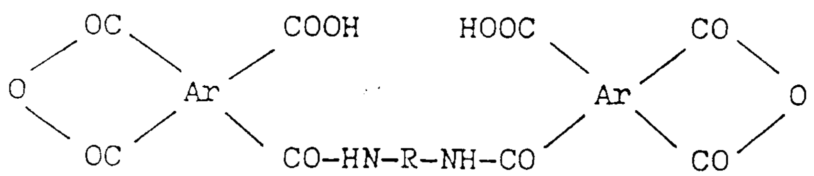

(VI)

entstehen, die gegebenenfalls chemisch oder thermisch zu den Tetracarbonsäuredianhydriden der allgemeinen Formel I, in der X den Imidrest der Formel III bedeutet, cyclisiert werden, worauf die gebildeten Tetracarbonsäuredianhydride der allgemeinen Formel I gegebenenfalls in an sich bekannter Weise mit Wasser, Alkoholen und/oder Salzsäure ganz oder teilweise zu den entsprechenden Säuren, Säurechloriden oder Estern der allgemeinen Formel I umgesetzt werden.

**2.** Verfahren gemäß Anspruch 1, dadurch gekennzeichnet, daß der Rest Ar einen Benzophenonrest darstellt.

**3.** Verfahren gemäß Anspruch 1 oder 2, dadurch gekennzeichnet, daß der Rest Ar einen Benzolrest darstellt.

**4.** Verfahren gemäß einem der Ansprüche 1 bis 3, dadurch gekennzeichnet, daß man als Tricarbonsäuremonoanhydridchlorid Trimellithsäureanhydridchlorid einsetzt.

**5.** Verfahren gemäß einem der Ansprüche 1 bis 4, dadurch gekennzeichnet, daß man als Tetracarbonsäuredianhydrid Pyromellithsäuredianhydrid einsetzt.

**6.** Verfahren gemäß einem der Ansprüche 1 bis 5, dadurch gekennzeichent, daß man als Tetracarbonsäuredianhydrid Benzophenontetracarbonsäuredianhydrid einsetzt.

## Claims

### Claims for the following Contracting States : AT, BE, CH, DE, FR, GB, GR, IT, LI, LU, NL, SE

1. Aromatic tetracarboxylic acids or derivatives thereof of the general formula I

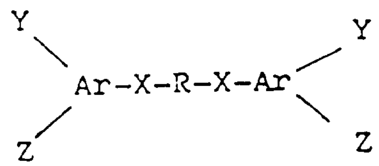

(I)

in which R denotes a divalent radical of the formula II

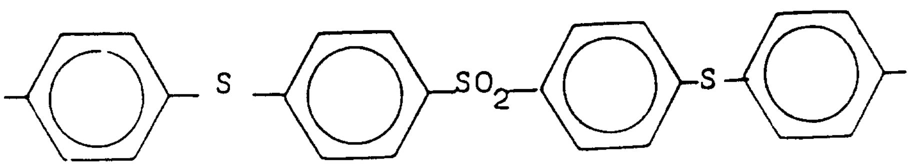

(II)

Ar denotes tri- or tetravalent aromatic radicals which are optionally substituted by one or more halogen atoms, or mixtures thereof, it additionally being possible for the trivalent aromatic radical optionally to contain a carboxyl group as a substituent, X, if Ar is trivalent, denotes the radical -CO-NH-, and if Ar is tetravalent denotes the imide radical of the formula III

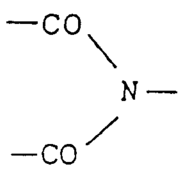

(III)

in which N is in each case bonded to R, and Y and Z either together denote the anhydride radical -CO-O-CO- or by themselves and independently of one another denote the radicals -COOH, -COCl or $-COOR_1$, in which $R_1$ represents an alkyl radical having 1 to 20 C atoms or an aryl radical having 6 to 20 C atoms.

2. Aromatic tetracarboxylic acids or derivatives thereof according to Claim 1, characterised in that the radical Ar represents a benzophenone radical.

3. Aromatic tetracarboxylic acids or derivatives thereof according to Claim 1 or 2, characterised in that the radical Ar represents a benzene radical.

4. Process for the preparation of aromatic tetracarboxylic acids or derivatives thereof according to one of Claims 1 to 3, characterised in that tricarboxylic acid monoanhydride chlorides or tetracarboxylic acid dianhydrides or mixtures thereof of the general formula IV

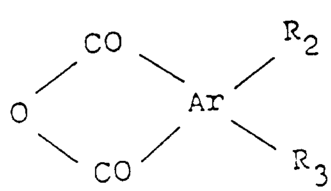

(IV)

in which, in the case of the tricarboxylic acid monoanhydride chlorides, $R_2$ stands for H and $R_3$ stands for -COCl, and in the case of the tetracarboxylic acid dianhydrides, $R_2$ and $R_3$ together stand for the

anhydride radical -CO-O-CO-, are reacted with the diamine of the general formula V

$H_2N$-R-$NH_2$ (V)

in which R has the above meaning, in a molar ratio of at least 2 : 1 in a manner which is known per se, the tetracarboxylic acid dianhydrides of the formula I in which X denotes the radical -CO-NH- being formed in the case where the tricarboxylic acid monoanhydride chlorides of the general formula IV are used, and the bis-amide acid dianhydrides of the general formula VI

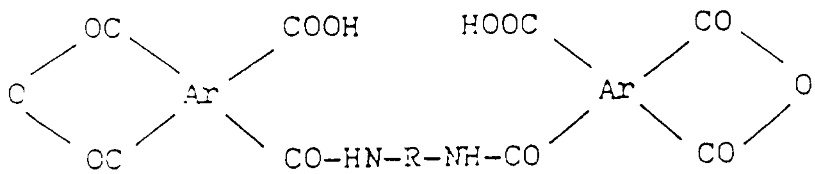

(VI)

initially being formed in the case where the tetracarboxylic acid dianhydrides of the general formula IV are used, these products, if appropriate, are cyclised chemically or by means of heat to give the tetracarboxylic acid dianhydrides of the general formula I in which X denotes the imide radical of the formula III and subsequently, if appropriate, the tetracarboxylic acid dianhydrides formed, of the general formula I, are completely or partly reacted with water, alcohols and/or hydrochloric acid in a manner which is known per se to give the corresponding acids, acid chlorides or esters of the general formula I.

**5.** Process according to Claim 4, characterised in that trimellitic acid anhydride chloride is used as the tricarboxylic acid monoanhydride chloride.

**6.** Process according to Claim 4 or 5, characterised in that pyromellitic acid dianhydride is used as the tetracarboxylic acid dianhydride.

**7.** Process according to one of Claims 4 to 6, characterised in that benzophenonetetracarboxylic acid dianhydride is used as the tetracarboxylic acid dianhydride.

**Claims for the following Contracting State: ES**

**1.** Process for the preparation of aromatic tetracarboxylic acids or derivatives thereof of the general formula I

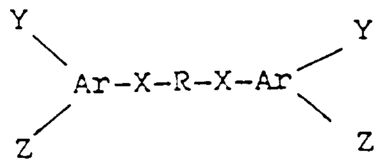

(I)

in which R denotes a divalent radical of the formula II

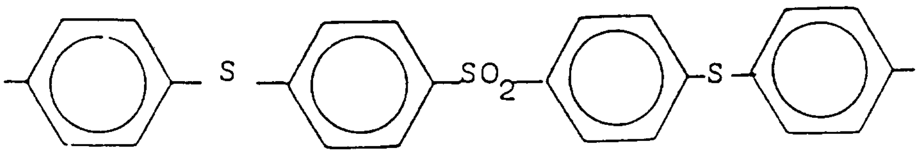

(II)

Ar denotes tri- or tetravalent aromatic radicals, which are optionally substituted by one or more halogen atoms, or mixtures thereof, it additionally being possible for the trivalent aromatic radical optionally to

contain a carboxyl group as a substituent, X, if Ar is trivalent, denotes the radical -CO-NH-, and if Ar is tetravalent denotes the imide radical of the formula III

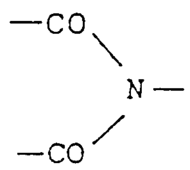

(III)

in which N is in each case bonded to R, and Y, Z either together denote the anhydride radical -CO-O-CO- or by themselves and independently of one another denote the radicals -COOH, -COCl or -COOR$_1$, in which R$_1$ represents an alkyl radical having 1 to 20 C atoms or an aryl radical having 6 to 20 C atoms, characterised in that tricarboxylic acid monoanhydride chlorides or tetracarboxylic acid dianhydrides or mixtures thereof of the general formula IV

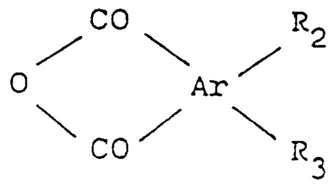

(IV)

in which, in the case of the tricarboxylic acid monoanhydride chlorides, R$_2$ stands for H and R$_3$ stands for -COCl, and in the case of the tetracarboxylic acid dianhydrides, R$_2$ and R$_3$ together stand for the anhydride radical -CO-O-CO-, are reacted with the diamine of the general formula V

$H_2N$-R-$NH_2$ (V)

in which R has the above meaning, in a molar ratio of at least 2 : 1 in a manner which is known per se, the tetracarboxylic acid dianhydrides of the formula I in which X denotes the radical -CO-NH- being formed in the case where the tricarboxylic acid monoanhydride chlorides of the general formula IV are used, and the bis-amide acid dianhydrides of the general formula VI

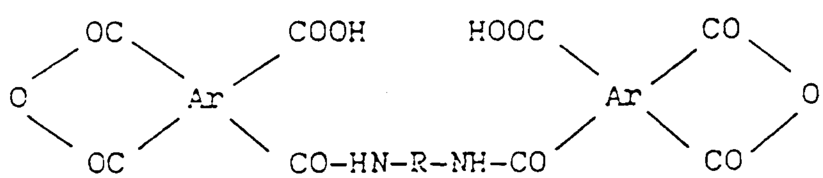

(VI)

initially being formed in the case where the tetracarboxylic acid dianhydrides of the general formula IV are used, these products, if appropriate, are cyclised chemically or by means of heat to give the tetracarboxylic acid dianhydrides of the general formula I in which X denotes the imide radical of the formula III and subsequently, if appropriate, the tetracarboxylic acid dianhydrides formed, of the general formula I, are completely or partly reacted with water, alcohols and/or hydrochloric acid in a manner which is known per se to give the corresponding acids, acid chlorides or esters of the general formula I.

2. Process according to Claim 1, characterised in that the radical Ar represents a benzophenone radical.

3. Process according to Claim 1 or 2, characterised in that the radical Ar represents a benzene radical.

4. Process according to one of Claims 1 to 3, characterised in that trimellitic acid anhydride chloride is used as the tricarboxylic acid monoanhydride chloride.

**5.** Process according to one of Claims 1 to 4, characterised in that pyromellitic acid dianhydride is used as the tetracarboxylic acid dianhydride.

**6.** Process according to one of Claims 1 to 5, characterised in that benzophenonetetracarboxylic acid dianhydride is used as the tetracarboxylic acid dianhydride.

**Revendications**
**Revendications pour les Etats contractants suivants : AT, BE, CH, DE, FR, GB, GR, IT, LI, LU, NL, SE**

**1.** Acides aromatiques tétracarboxyliques ou leurs dérivés de formule générale I

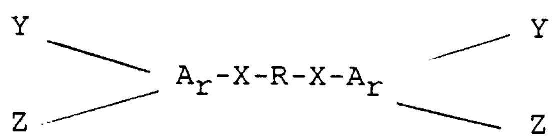

dans laquelle :

R signifie un reste divalent de formule II

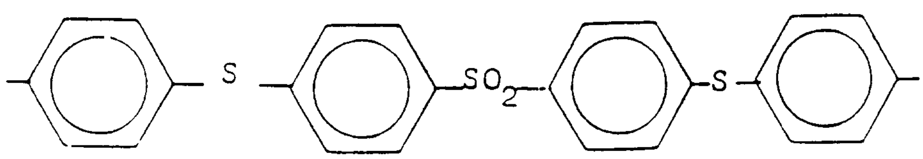

$A_r$ signifie des restes aromatiques tri- ou tétravalents, substitués le cas échéant par un ou plusieurs halogènes ou bien un mélange de ceux-ci, le reste aromatique trivalent renfermant comme substituant le cas échéant un groupe carboxylique supplémentaire,

X dans le cas où $A_r$ est trivalent, désigne le reste -CO-NH-, et dans le cas où $A_r$ est tétravalent, le reste imide de formule III

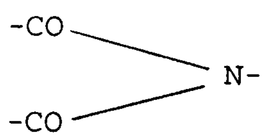

où N est toujours lié à R,

Y,Z signifient soit ensemble le reste anhydride -CO-O-CO-, ou bien seuls et indépendants l'un de l'autre les restes - COOH, -COCl ou -$COOR_1$ où $R_1$ représente un reste alkyle de 1 à 20 atomes de carbone ou un reste aryle de 6 à 20 atomes de carbone.

**2.** Acides aromatiques tétarcarboxyliques ou leurs dérivés selon la revendication 1, caractérisés en ce que le reste $A_r$ représente un reste benzophénone.

**3.** Acides aromatiques tétracarboxyliques ou leurs dérivés selon la revendication 1 ou 2, caractérisés en ce que le reste $A_r$ représente un reste benzénique.

**4.** Procédé de préparation des acides aromatiques tétracarboxyliques ou de leurs dérivés selon une des revendications 1 à 3, caractérisé en ce qu'on fait réagir les chlorures monoanhydrides tricarboxyliques ou dianhydrides tétracarboxyliques ou leur mélange de formule générale IV

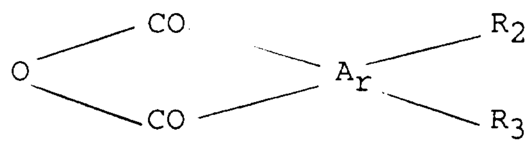

dans le cas du chlorure monoanhydride de l'acide tricarboxylique $R_2$ est H et $R_3$ COCl, et dans le cas du dianhydride de l'acide tétracarboxylique $R_2$ et $R_3$ représentent ensemble le reste anhydride -CO-O-CO-, avec la diamine de formule générale V.

$H_2N-R-NH_2$

dans laquelle R a la signification sus-indiquée, en proportion molaire d'au moins 2:1, de manière connue en soi, où on obtient dans le cas de l'utilisation des chlorures monoanhydride de l'acide tricarboxylique de formule générale IV les dianhydrides de l'acide tétracarboxylique de formule générale I dans laquelle X signifie le reste -CO-NH, et où on obtient dans le cas de l'utilisation des dianhydrides de l'acide tétracarboxylique de formule générale IV d'abord les dianhydrides de l'acide bis-amide de formule générale VI

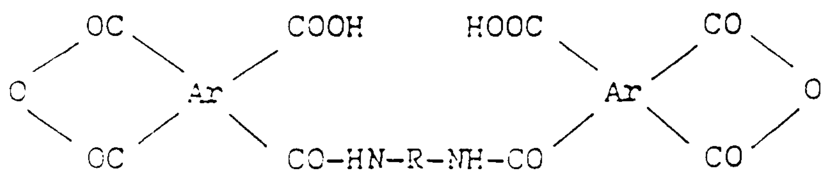

qui sont rendus cycliques chimiquement ou thermiquement en dianhydrides tétracarboxyliques de formule générale I dans laquelle X signifie le reste imide de formule III, les dianhydrides de l'acide tétracarboxylique formés de formule générale I étant transformés le cas échéant de manière connue avec de l'eau, des alcools, et/ou l'acide chlorhydrique totalement ou partiellement en acides, chlorures acides, ou esters correspondants de formule générale I.

5. Procédé selon la revendication 4, caractérisé en ce qu'on utilise comme chlorure monoanhydride de l'acide tricarboxylique le chlorure anhydride de l'acide trimellitique.

6. Procédé selon la revendication 4 ou 5, caractérisé en ce qu'on utilise comme dianhydride de l'acide tétracarboxylique le dianhydride de l'acide pyromellitique.

7. Procédé selon l'une des revendications 4 à 6, caractérisé en ce qu'on utilise comme dianhydride de l'acide tétracarboxylique le dianhydride de l'acide benzophénonetétracarboxylique.

**Revendications pour l'Etat contractant suivant : ES**

1. Procédé de préparation des acides aromatiques tétracarboxyliques ou de leurs dérivés de formule générale I

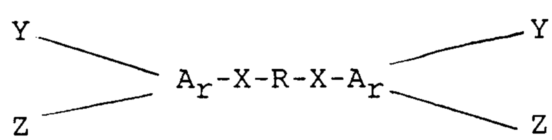

dans laquelle :

R signifie un reste divalent de formule II

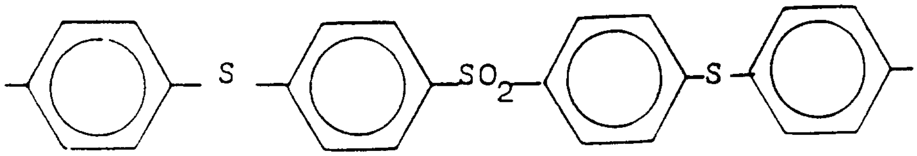 (II)

- $A_r$ signifie des restes aromatiques tri- ou tétravalents, substitués le cas échéant par un ou plusieurs halogènes ou bien un mélange de ceux-ci, le reste aromatique trivalent renfermant comme substituant le cas échéant un groupe carboxylique supplémentaire,
- X dans le cas où $A_r$ est trivalent, désigne le reste -CO-NH-, et dans le cas où $A_r$ est tétravalent, le reste imide de formule III

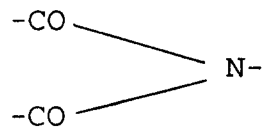

où N est toujours lié à R,

- Y,Z signifient soit ensemble le reste anhydride -CO-O-CO-, ou bien seuls et indépendants l'un de l'autre les restes - COOH, -COCl ou -$COOR_1$ où $R_1$ représente un reste alkyle de 1 à 20 atomes de carbone ou un reste aryle de 6 à 20 atomes de carbone.

caractérisé en ce qu'on fait réagir les chlorures monoanhydrides tricarboxyliques ou dianhydrides tétracarboxyliques ou leur mélange de formule générale IV

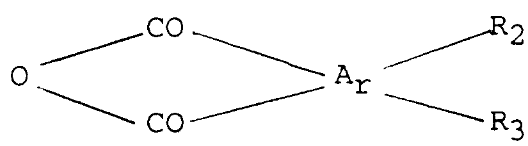

dans le cas du chlorure monoanhydride de l'acide tricarboxylique $R_2$ est H et $R_3$ COCl, et dans le cas du dianhydride de l'acide tétracarboxylique $R_2$ et $R_3$ représentent ensemble le reste anhydride -CO-O-CO-, avec la diamine de formule générale V.

$H_2N$-R-$NH_2$

dans laquelle R a la signification sus-indiquée, en proportion molaire d'au moins 2:1, de manière connue en soi, où on obtient dans le cas de l'utilisation des chlorures monoanhydrides de l'acide tricarboxylique de formule générale IV les dianhydres de l'acide tétracarboxylique de formule I dans laquelle X signifie le reste -CO-NH, et où on obtient dans le cas de l'utilisation des dianhydrides de l'acide tétracarboxylique de formule générale IV d'abord les dianhydrides de l'acide bis-amide de formule générale VI

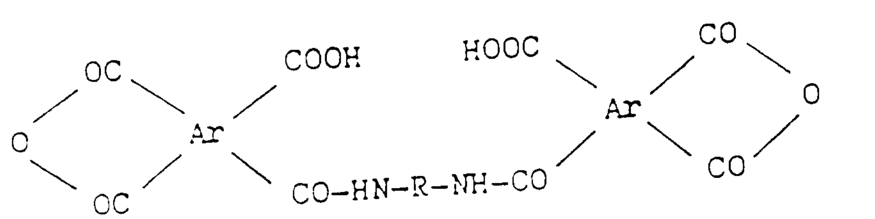 (VI)

qui sont rendus cycliques chimiquement ou thermiquement en dianhydrides tétracarboxyliques de formule générale I dans laquelle X signifie le reste imide de formule III, les dianhydrides de l'acide tétracarboxylique formés de formule générale I étant transformés le cas échéant de manière connue avec de l'eau, des alcools, et/ou l'acide chlorhydrique totalement ou partiellement en acides, chlorures acides, ou esters correspondants de formule générale I.

2. Procédé selon la revendication 1, caractérisé en ce que le reste $A_2$ représente un reste benzophénone.

3. Procédé selon la revendication 1 ou 2, caractérisé en ce que le reste $A_2$ représente un reste benzénique.

4. Procédé selon l'une des revendications 1 à 3, caractérisé en ce qu'on utilise comme chlorure monoanhydride de l'acide tricarboxylique le chlorure anhydride de l'acide trimellitique.

5. Procédé selon l'une des revendications 1 à 4, caractérisé en ce qu'on utilise le dianhydride de l'acide pyromellitique comme dianhydride de l'acide tétra carboxylique.

6. Procédé selon l'une des revendications 1 à 5, caractérisé en ce qu'on utilise le dianhydride de l'acide benzophénonetétracarboxylique comme dianhydride de l'acide tétracarboxylique.

Formelblatt

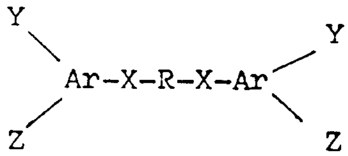

I

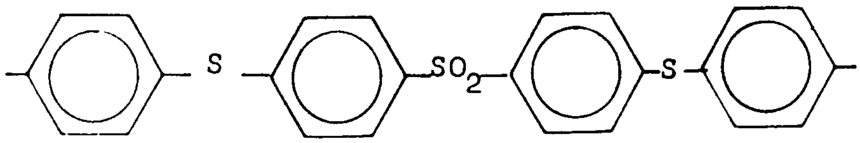

II

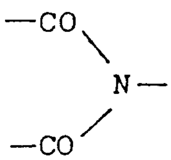

III

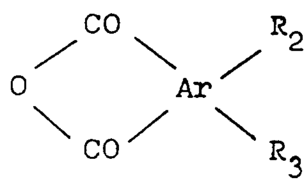

IV

$H_2N-R-NH_2$

V

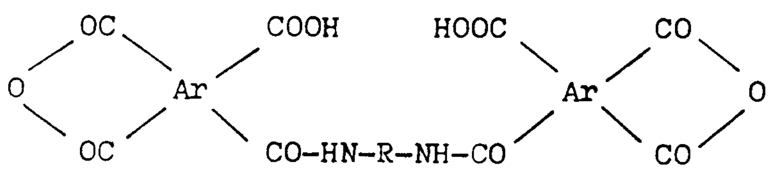

VI

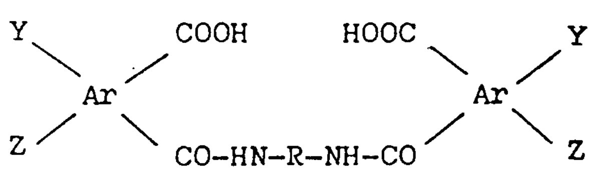

VII